(19) **Europäisches Patentamt European Patent Office Office européen des brevets**

(11) **EP 2 949 748 B1**

(12) **EUROPEAN PATENT SPECIFICATION**

(45) Date of publication and mention of the grant of the patent:
**27.02.2019 Bulletin 2019/09**

(21) Application number: **15175933.9**

(22) Date of filing: **29.05.2006**

(51) Int Cl.:
*C12N 9/24* (2006.01)     *C12N 9/82* (2006.01)
*A21D 8/04* (2006.01)

(54) **NOVEL PROCESS FOR ENZYMATIC ACRYLAMIDE REDUCTION IN FOOD PRODUCTS**

NEUARTIGES VERFAHREN ZUR ENZYMATISCHEN ACRYLAMIDREDUKTION IN LEBENSMITTELPRODUKTEN

NOUVEAU PROCÉDÉ DE RÉDUCTION ENZYMATIQUE D'ACRYLAMIDE DANS DES PRODUITS ALIMENTAIRES

(84) Designated Contracting States:
**AT BE BG CH CY CZ DE DK EE ES FI FR GB GR HU IE IS IT LI LT LU LV MC NL PL PT RO SE SI SK TR**

(30) Priority: **31.05.2005 EP 05104683**

(43) Date of publication of application:
**02.12.2015 Bulletin 2015/49**

(62) Document number(s) of the earlier application(s) in accordance with Art. 76 EPC:
**14168497.7 / 2 767 586**
**06763331.3 / 1 896 576**

(73) Proprietor: **DSM IP Assets B.V.**
**6411 TE Heerlen (NL)**

(72) Inventor: **DE BOER, Lex**
**6100 AA Echt (NL)**

(74) Representative: **DSM Intellectual Property**
**P.O. Box 4**
**6100 AA Echt (NL)**

(56) References cited:
**WO-A-03/083043      WO-A-2004/026043**
**WO-A-2004/030468      WO-A-2004/032648**
**WO-A-2004/037007      US-A1- 2003 180 418**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

This disclosure relates to a novel enzyme composition suitable for use in a food preparation process in order to decrease acrylamide content in food products.

The novel enzyme composition is especially suitable for use in baking industry.

Recently, the occurrence of acrylamide in a number of food and oven prepared foods was published (Tareke et al. Chem. Res. Toxicol. 13, 517-522 (2000). Since acrylamide is considered as probably carcinogenic for animals and humans, this finding had resulted in world-wide concern. Further research revealed that considerable amounts of acrylamide are detectable in a variety of baked, fried and oven prepared common foods and it was demonstrated that the occurrence of acrylamide in food was the result of the baking process.

The official limit in the UK for acrylamide contamination in food products is set at 10 ppb (10 micrograms per kilogram) and the values presented above abundantly exceed this value for a lot of products, especially cereals, bread products and potato or corn based products.

A pathway for the formation of acrylamide from amino acids and reducing sugars as a result of the Maillard reaction has been proposed by Mottram et al. Nature 419:448 (2002). According to this hypothesis, acrylamide may be formed during the Maillard reaction. During baking and roasting, the Maillard reaction is mainly responsible for the color, smell and taste. A reaction associated with the Maillard is the Strecker degradation of amino acids and a pathway to acrylamide was proposed. The formation of acrylamide became detectable when the temperature exceeded 120 °C, and the highest formation rate was observed at around 170 °C. When asparagine and glucose were present, the highest levels of acrylamide could be observed, while glutamine and aspartic acid only resulted in trace quantities. The fact that acrylamide is formed mainly from asparagine (combined with reducing sugars) may explain the high levels acrylamide in oven-cooked or roasted plant products. Several plant raw materials are known to contain substantial levels of asparagine. In potatoes asparagine is the dominant free amino acid (940 mg/kg, corresponding with 40% of the total amino-acid content) and in wheat flour asparaginase is present as a level of about 167mg/kg, corresponding with 14% of the total free amino acids pool (Belitz and Grosch in Food Chemistry - Springer New York, 1999). Therefore, in the interest of public health, there is an urgent need for food products that have substantially lower levels of acrylamide or, preferably, are devoid of it.

A variety of solutions to decrease the acrylamide content has been proposed, either by altering processing variables, e.g. temperature or duration of the heating step, or by chemically or enzymatically preventing the formation of acrylamide or by removing formed acrylamide. The present invention involves enzymatic decrease of formation of acrylamide.

Enzymatic routes to decrease the formation of acrylamide are amongst others the use of asparaginase to decrease the amount of asparagine in the food product, since asparagine is seen as an important precursor for acrylamide.

WO2004/037007 describes an enzymatic composition comprising asparaginase and cellulose and the use thereof to decrease acrylamide levels in roasted coffee beans.

WO2003/083043 describes recombinant methods for producing a secreted polypeptide having L-asparaginase activity, as well as the isolated polypepitdes having L-asparaginase activity and nucleic acids thereof.

WO2004/030468 describes a process for production of a food product like batard breads involving a heating step, wherein dough is treated with asparaginase and subsequently the dough was baked.

WO2004/032648 describes a method of preparing a heat treated product, comprising providing a raw material, like a dough, treating the raw material with asparaginase, and heat treating to reach a final water content below 35 % by weight.

WO2004/026043 describes a method for reducing the level of asparagine in a food material like potato or corn comprising adding an asparagine reducing enzyme to the food material before heating.

However, for some applications the use of asparaginase alone is not sufficient to decrease the acrylamide content of the food product to the desired level. Therefore, it is the object of the present invention to provide an enzyme composition resulting in an improved decrease of acrylamide levels in food prepared by use of the composition according to the disclosure.

The objective of the present invention is reached by providing an enzyme composition comprising asparaginase and xylanase.

Surprisingly, it was found that the addition of xylanase together with asparaginase results in a synergetic effect with respect to decrease acrylamide levels in food prepared with this enzyme composition.

An enzyme composition comprising asparaginase and an enzyme capable of oxidizing the reducing sugars is disclosed in WO 2004/032648 as is in line with the teaching that acrylamide is formed by the reaction between asparagine and reducing sugars.

However, the enzyme composition according to the present disclosure increases the amount of reducing sugars, but still reaches a dramatic decrease in the acrylamide level of the food product, even lower than when only asparaginase would have been added.

Any asparaginase (EC 3.5.1.1) available can be used in the present invention. Suitable asparaginase (E.C. 3.5.1.1) can be obtained from various sources, such as for Example from plants, animals and microorganisms. Examples of suitable microorganisms are *Escheria*, *Erwinia*, *Streptomyces*, *Pseudomonas*, *Aspergillus* and *Baccillus* species. Examples of suitable asparaginases can be found in WO03/083043 and WO2004/030468. A preferred asparaginase is the asparaginase having SEQ ID NO:3 or a functional equivalent thereof as described in WO04/030468.

Any hydrolyzing enzyme (EC 3.x.x.x) can be suitable for the present disclosure. For the EC classification references as made herein the Recommended Enzyme Nomenclature (1992) of the IUBMB published by Academic Press Inc. (ISBN 0-12-227165-3) were used. X is herein used to indicate an integer.

However, preferably the hydrolyzing enzymes are used which belong to the group of carboxylic ester hydrolases (EC 3.1.1.x) or from the group of glycosidases hydrolyzing o-glycosyl compounds (EC 3.2.1.x.).

Examples of suitable carboxylic ester hydrolases are lipases (EC 3.1.1.3), pectin esterase (EC 3.1.1.11), galactolipase EC 3.1.1.26), phospholipase A1 (EC 3.1.1.32), phospholipase A2 (EC 3.1.1.4), lysophospholipase (EC 3.1.1.5).

Examples of preferred suitable hydrolysing o-glycosyl compounds are alpha-amylase (EC 3.2.1.1), beta-amylase (EC 3.2.1.2), pectinase (EC 3.2.1.15), cellulase (EC 3.2.1.4), xylanase (EC 3.2.1.32), arabinofuranosidase (EC 3.2.1.55), and glucanase (EC 3.2.1.6).

Also mixtures of hydrolyzing enzymes may be used in the composition according to the disclosure, including mixtures of carboxylic ester hydrolases with hydrolyzing o-glycosyl compounds. The person skilled in the art knows how to obtain the hydrolysing enzymes suitable for use in the disclosure.

In the present invention, asparaginase is combined with xylanase. This composition is especially suitable for baking industry and might be part of a pre-mix.

In another embodiment, asparaginase is combined with an enzyme which allows the mobilization of the asparaginase or the penetration of the asparaginase. These compositions are especially suitable when structurally intact cells of plant origin are present and an endogenous polymer of the plant matrix has to be hydrolysed.

In a second aspect of the invention, the invention relates to a novel process to reduce acrylamide content in food products. In one preferred embodiment, the food product is a baked product. In another preferred embodiment, the food product is a deep-fried product. In yet another preferred embodiment, the food product is a roasted or toasted product, in particular a roasted or toasted dough or bread.

The process for the production of a food product involving at least one heating step comprises adding asparaginase and at least one hydrolyzing enzyme to an intermediate form of said food product in said production process whereby the asparaginase and at least one hydrolyzing enzyme are added prior to said heating step in an amount that is effective in reducing the level of acrylamide of the food product in comparison to a food product whereto no asparaginase and hydrolyzing enzyme were added, wherein the hydrolyzing enzyme is a xylanase.

The asparaginase and at least one hydrolyzing enzyme can be added separately or in a composition, preferably in a composition according to the disclosure. Preferably, the composition is added to the food production process in an amount that the acrylamide content of the food product produced in the presence of the enzyme composition according to the disclosure is decreased relative to a food product produced without either one of the components in the composition according to the .

More preferably, the composition is added to the food production process in an amount that the acrylamide content of the food product produced in the presence of the enzyme is reduced by at least 10%, 15%, 20%, 25% or 30%, preferably by at least 35%, 40%, 45%, 50%, 55%, 60%, 65% or 70%, more preferably by at least 80%, 85% or 90%, most preferably by at least 95%, 97%, 98% or 99% as compared to food produced in the presence of asparaginase and in the absence of the hydrolyzing enzyme. For the asparaginase and the hydrolyzing enzyme to be used in the method according to the invention, the same preferences are to be considered as described above.

An intermediate form of the food product is defined herein as any form that occurs during the production process prior to obtaining the final form of the food product, this includes parts of plants, but also a slice or a cut of a plant part. The intermediate form may comprise the individual raw materials used and/or processed form thereof. To give just two Examples, for the food product bread, the intermediate forms can comprise wheat, wheat flour, the initial mixture thereof with other bread ingredients, such as for Example water, salt, yeast and bread improving compositions, the mixed dough, the kneaded dough, the frozen dough, the leavened dough and the partially baked dough. For the food product shaped potato chips, the intermediate forms can comprise boiled potato, mashed potato, dried mashed potato and potato dough.

The food product may be made from at least one raw material that is of plant origin, for Example potato, tobacco, coffee, cocoa, rice, cereal, fruit. Examples of cereals are wheat, rye, corn, maize, barley, groats, buckwheat and oat. Wheat is here and hereafter intended to encompass all known species of the Triticum genus, for Example aestivum, durum and/or spelta. Also food products made from more than one raw material are included in the scope of this invention, for Example food products comprising both wheat (flour) and potato.

Examples of food products in which the process according to the invention can be suitable for are any flour based products - for Example bread, pastry, cake, pretzels, bagels, Dutch honey cake, cookies, gingerbread, gingercake and crispbread -, and any potato-based products - for Example French fries, pommes frites, potato chips, croquettes - and any corn-base product - for Example corn bread, corn crisps and corn flakes.

A preferred production process is the baking of bread and other baked products from wheat flour and/or flours from other cereal origin. Another preferred production process is the deep-frying of potato chips from potato slices. Still another preferred production process is the deep-frying of corn crisps from extruded corn based dough.

Preferred heating steps are those at which at least a part of the intermediate food product, e.g. the surface of the food product, is exposed to temperatures at which acrylamide formation is promoted, e.g. 110 °C or higher, 120 °C or higher. The heating step in the process according to the invention may be carried out in ovens, for instance at a temperature between 180-220 °C, such as for the baking of bread and other bakery products, or in oil such as the frying of potato chips, for Example at 160-190 °C.

The invention is hereafter illustrated by the following non-limiting Examples.

**Short description of the figures**

Figure 1 The effect of 50 ppm asparaginase in several enzyme combinations on acrylamide levels in crusts of mini-batards prepared with leavening salts (in %). The acrylamide level of the enzyme combination without asparaginase was set at 100%.

Figure 2 The effect of 50 ppm *A. niger* asparaginase in several enzyme combinations on acrylamide levels in crusts of mini-batards prepared with with Mogul Brand Chapatti brown flour and baker's yeast. The acrylamide level of the enzyme combination without asparaginase was set at 100% .

Figure 3 The effect of *A. niger* asparaginase in several enzyme combinations on acrylamide levels in crusts of mini-batards prepared with kolibri flour and baker's yeast. The acrylamide level of bread with asparaginase as the sole baking enzyme was set at 100%.

**Materials**

*Table 1. Used baking enzymes in the Examples*

| Baking enzyme | Enzyme activity | Supplier |
|---|---|---|
| Bakezyme P500 | Alpha-amylase | DSM Food Specialties |
| Bakezyme HSP6000 | Xylanase | DSM Food Specialties |
| Bakezyme W | Glucanase/cellulase | DSM Food Specialties |
| Bakezyme XE | Cellulase | DSM Food Specialties |
| Bakezyme A | Alpha-L-arabinofuranoside arabinofuranohydrolase/ Arabinofuranosidase A | DSM Food Specialties |
| Lipopan F | Galactolipase/phospholipase A1 / phospholipase A2/ Lysophospholipase/lipase | Novozymes A/S |

**Example 1**

**Acrylamide measurement**

**Sample pretreatment**

600 mg dried and homogenized sample is extracted using 5 ml of milliQ water. 1 μg of internal standard $^{13}C_3$

acrylamide in solution (CIL) is added to the extract. After 10 minutes of centrifugation (6000 rpm), 3 ml of the upper layer is brought on an Extreluut-3BT column (Merck). Using 15 ml of ethylacetate, acrylamide is eluted from the column. Ethylacetate is evaporated under a gentle stream of nitrogen down to approximately 0.5 ml.

**Chromatographic conditions**

The ethylacetate solution is analysed using gas chromatography. Separation is obtained using a CP-Wax 57 (Varian) column (length 25 m, internal diameter 0.32 mm, film 1.2 $\mu$m) and helium as the carrier gas with a constant flow of 5.4 ml/min. Split-less injection of 3 $\mu$l is performed. Oven temperature is kept at 50°C for 1 minute, after which the temperature is increased with 30°C/min towards 220° C. After 12 minutes of constant temperature of 220°C the oven is cooled down and stabilized before next injection.

Detection is performed using on-line chemical ionization mass spectrometry in positive ion mode, using methane as ionization gas. The characteristic ions m/z 72 (acrylamide) and m/z 75 ($^{13}C_3$ acrylamide) are monitored for quantification.

**Used Equipment**

|  |  |
|---|---|
| GC: | HP6890 (Hewlet Packard) |
| MSD (mass selective detector): | HP5973 (Hewlet Packard) |

Amounts in ppm or ppb are based on the amount of flour, unless stated otherwise.

**Example 2**

**Effects of baking enzymes and *Aspergillus niger* asparaginase on acrylamide formation in mini-batard breads prepared with leavening salts**

Preparation of minibatards with leavening salts was done by mixing 200 g whole-wheat flour (Mogul Brand Chapatti brown flour, Mogul Lasu B.V. The Hague, Holland), 4 g salt, 68 ppm ascorbic acid, 2 g DKS (NaHCO$_3$)(Chem Proha, Chemiepartners B.V. Dordrecht, Holland), 2.7 g Sap 40 (Sodium acid pyrophosphate, E450) (Chemische Fabrik Budenheim KG, Budenheim, Germany) 1 g SSL (Sodium stearoyl lactylate)(Danisco, Denmark) 1 g GMS (glyceryl mono stearate, (Admul), Quest, Naarden, Holland) Amounts of baking enzymes to be tested are indicated in Table 1 (Lipopan F and Novamyl are obtainable from Novo, the other enzymes are obtainable from DSM-Gist). 226 ml water was added. Mixing took place in a pin mixer for 8 minutes and 45 seconds. The dough temperature was 27°C. Directly after mixing the dough is divided into two pieces of 150 g, rounded and proofed for 25 minutes in a proofing cabinet at 32°C. Hereafter the dough pieces were shaped and a final proof was performed at 32°C for 100 minutes. The dough pieces were baked for 20 minutes at 225°C. The acrylamide in the crust was determined as is described in Example 1. The percentage acrylamide that was left in the asparaginase treated breads was calculated as follows:

$$\frac{\text{acrylamide content (baking enzyme combination X plus asparaginase)}}{\text{acrylamide content (baking enzyme combination X without asparaginase)}} \times 100\%$$

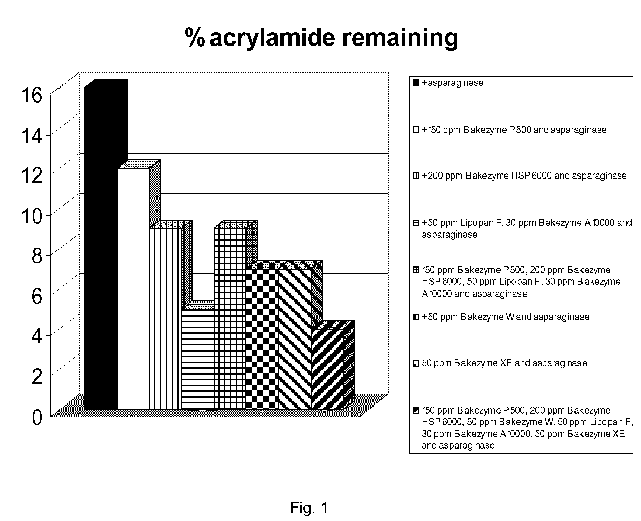

and is shown in Table 2 and Figure 1 for several enzyme combinations. For Example, the percentage acrylamide remaining in bread treated with Bakezyme P500 and asparaginase was calculated by dividing the results from test no. 4 by the results from test no. 3 and multiplying this by 100%.

*Table 2. Acrylamide in crusts of mini-batard breads prepared with leavening salts and several baking enzymes as is indicated in the Example and the effect of Aspergillus niger asparaginase on acrylamide levels.*

| Test no. | Baking Enzyme | Dosage (ppm) | Acrylamide content (ppb) | % acrylamide remaining |
|---|---|---|---|---|
| 1 | None |  | 185 | 100 |
| 2 | Asparaginase | 50 | 30 | 16 |

(continued)

| Test no. | Baking Enzyme | Dosage (ppm) | Acrylamide content (ppb) | % acrylamide remaining |
|---|---|---|---|---|
| 3 | Bakezyme P500 | 150 | 143 | 100 |
| 4 | Bakezyme P500 Asparaginase | 150 50 | 17 | 12 |
| 5 | Bakezyme HSP6000 | 200 | 234 | 100 |
| 6 | Bakezyme HSP6000 Asparaginase | 200 50 | 21 | 9 |
| 7 | Lipopan F Bakezyme A10000 | 50 30 | 250 | 100 |
| 8 | Lipopan F Bakezyme A10000 Asparaginase | 50 30 50 | 13 | 5 |
| 9 | Bakezyme P500 Bakezyme HSP6000 Lipopan F bakezyme A10000 | 150 200 50 30 | 279 | 100 |
| 10 | Bakezyme P500 Bakezyme HSP6000 Lipopan F Bakezyme A10000 asparaginase | 150 200 50 30 50 | 25 | 9 |
| 11 | Bakezyme W | 50 | 263 | 100 |
| 12 | Bakezyme W asparaginase | 50 50 | 19 | 7 |
| 13 | Bakezyme XE | 50 | 228 | 100 7 |
| 14 | Bakezyme XE asparaginase | 50 50 | 17 | |
| 15 | Bakezyme P500 Bakezyme HSP6000 Bakezyme W Lipopan F Bakezyme A10000 Bakezyme XE | 150 200 50 50 30 50 | 464 | 100 |
| 16 | Bakezyme P500 Bakezyme HSP6000 Bakezyme W Lipopan F Bakezyme A10000 Bakezyme XE asparaginase | 150 200 50 50 30 50 50 | 18 | 4 |

From Table 2 and Figure 1 it can be concluded that addition of the baking enzymes Bakezyme® HSP6000, Lipopan® F, Bakezyme® A10000, Bakezyme® W, Bakezyme® XE and combinations thereof, will result in an increased level of acrylamide in the crust compared to a reference bread without baking enzymes. Addition of an appropriate amount of asparaginase to the dough will however result in a decreased level of acrylamide compared to the corresponding reference without asparaginase and even lower than a reference in which no baking enzymes were used.

## Example 3

**Effects of baking enzymes and *A*. niger asparaginase on acrylamide formation in the mini-batards breads prepared with baking yeast and whole wheat flour**

Preparation of mini-batard breads in a standard baking process was done by mixing 200 g of whole-wheat flour (Mogul Brand Chapatti brown flour) 4.6 g Koningsgist® yeast, 4 g salt, 68 ppm ascorbic acid and several enzymes and enzyme combinations as indicated in Table 2. 132 g water was added and mixing was performed in a pin mixer for 8 minutes and 45 seconds. The dough temperature was 27°C. Directly after mixing the dough was divided into two pieces of 150 g, rounded and proofed for 25 minutes in a proofing cabinet at 32°C. Hereafter, the dough pieces were shaped and a final proof was performed of 100 minutes at 32°C, the dough pieces were baked for 20 minutes at 225°C. The acrylamide in the crust was determined as is described in Example 1. The percentage acrylamide that was left in the asparaginase treated breads was calculated as is indicated in Example 2.

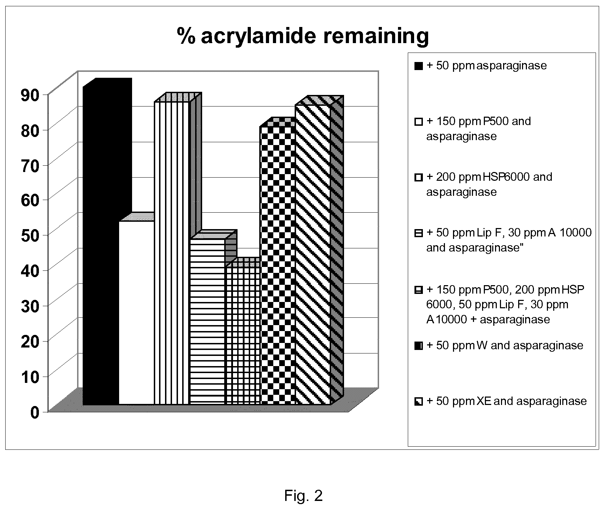

In Table 3 and Figure 2 the effects of asparaginase are shown in several enzyme combinations.

*Table 3. Acrylamide in crusts of mini-batard breads prepared with whole wheat flour, yeast and several baking enzymes and the effect of Aspergillus niger asparaginase on acrylamide levels.*

| Test no. | Baking Enzyme | Dosage (ppm) | Acrylamide content (ppb) | remaining% acrylamide |
|---|---|---|---|---|
| 1 | None | | 78 | 100 |
| 2 | asparaginase | 50 | 70 | 90 |
| 3 | Bakezyme P500 | 15 | 73 | 100 |
| 4 | Bakezyme P500 asparaginase | 15 50 | 65 | 89 |
| 5 | Bakezyme P500 | 150 | 94 | 100 |
| 6 | Bakezyme P500 Asparaginase | 150 50 | 49 | 52 |
| 7 | Bakezyme HSP6000 | 50 | 77 | 100 |
| 8 | Bakezyme HSP6000 Asparaginase | 50 50 | 67 | 87 |
| 9 | Bakezyme HSP6000 | 200 | 70 | 100 |
| 10 | Bakezyme HSP6000 Asparaginase | 200 50 | 60 | 86 |
| 11 | Lipopan F Bakezyme A10000 | 50 30 | 159 | 100 |
| 12 | Lipopan F Bakezyme A10000 Asparaginase | 50 50 50 | 74 | 47 |
| 13 | Bakezyme XE | 50 | 80 | 100 |
| 14 | Bakezyme XE Asparaginase | 50 50 | 68 | 85 |
| 15 | Bakezyme P500 Bakezyme HSP6000 Bakezyme A10000 Lipopan F | 150 200 30 50 | 257 | 100 |

(continued)

| Test no. | Baking Enzyme | Dosage (ppm) | Acrylamide content (ppb) | remaining% acrylamide |
|---|---|---|---|---|
| 16 | Bakezyme P500<br>Bakezyme HSP6000<br>Bakezyme A10000<br>Lipopan F<br>Asparaginase | 150<br>200<br>30<br>50<br>50 | 100 | 39 |
| 17 | Bakezyme W | 50 | 90 | 100 |
| 18 | Bakezyme W<br>Asparaginase | 50<br>50 | 71 | 79 |

In Figure 2 the effects are presented of *A. niger* asparaginase in the presence of (combinations) of enzymes. Compared to the acrylamide level in crust of breads prepared with the mentioned an enzyme or enzyme combination, the relative and in some cases even the absolute acrylamide levels are lower when asparaginase is used in the presence of (combinations) of enzymes.

From Table 3 and Figure 2 it can be concluded that addition of the baking enzymes Bakezyme P500, Bakezyme A10000, Bakezyme HSP6000, Lipopan F, Bakezyme W, Bakezyme XE and combinations thereof, will result in an increased level of acrylamide in the crust compared to a reference bread indifferent whether it is prepared with the leavening salt $NaHCO_3$ or yeast. Addition of an appropriate amount of asparaginase to the dough will however result in a decreased level of acrylamide compared to the corresponding reference without asparaginase and in some cases even lower than a reference in which no baking enzymes were used but where asparaginase was present.

## Example 4

**Effects of baking enzymes and *A. niger* asparaginase on acrylamide formation in the mini-batards breads prepared with baking yeast and kolibri flour**

Preparation of mini-batard breads in a standard baking process was done by mixing 200 g of kolibri flour (Meneba) 4.6 g Koningsgist® yeast, 4 g salt, 68 ppm ascorbic acid and several enzymes and enzyme combinations as indicated in Table 2. 114 g water was added and mixing was performed in a pin mixer for 6 minutes and 15 seconds. The dough temperature was 27°C. Directly after mixing the dough was divided into two pieces of 150 g, rounded and proofed for 25 minutes in a proofing cabinet at 32°C. Hereafter, the dough pieces were shaped and a final proof was performed of 100 minutes at 32°C, the dough pieces were baked for 20 minutes at 225°C. The acrylamide in the crust was determined as is described in Example 1. The percentage acrylamide that was left in the asparaginase treated breads was calculated as is indicated in Example 2.

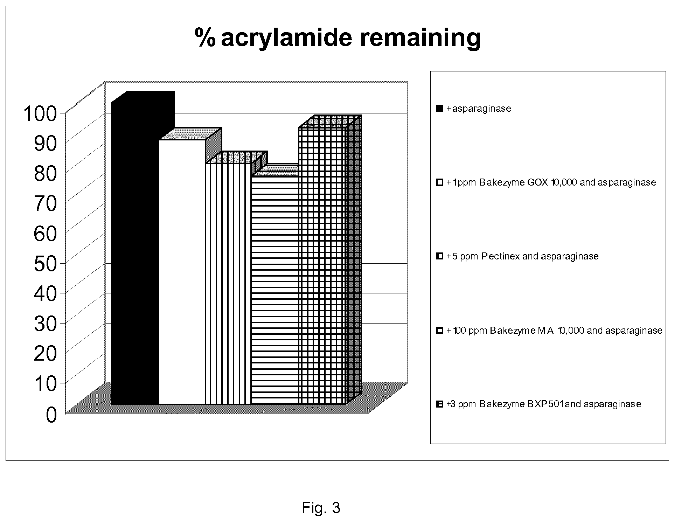

In Table 4 and Figure 3 the effects of asparaginase are shown in several enzyme combinations.

*Table 4. Acrylamide in crusts of mini-batard breads prepared kolibri flour with yeast and several baking enzymes and the effect of Aspergillus niger asparaginase on acrylamide levels.*

| Test no. | Baking Enzyme | Dosage (ppm) | Acrylamide content (ppb) | remaining% acrylamide |
|---|---|---|---|---|
| 1 | None | | 50 | 100 |
| 2 | asparaginase | 50 | 42 | 84 |
| 3 | Bakezyme GOX 10,000 | 1 | 40 | 100 |
| 4 | Bakezyme GOX 10,000<br>asparaginase | 1<br>50 | 37 | 93 |
| 5 | Pectinex * | 5 | 41 | 100 |
| 6 | Pectinex<br>Asparaginase | 5<br>50 | 34 | 83 |
| 7 | Bakezyme MA 10,000 | 100 | 48 | 100 |

(continued)

| Test no. | Baking Enzyme | Dosage (ppm) | Acrylamide content (ppb) | remaining% acrylamide |
|---|---|---|---|---|
| 8 | Bakezyme MA 10,000 Asparaginase | 100 50 | 32 | 67 |
| 9 | Bakezyme BXP501 | 3 | 43 | 100 |
| 10 | Bakezyme BXP501 Asparaginase | 3 50 | 39 | 91 |
| *: Pectinex is derived from NOVO. | | | | |

In Figure 3 the effects are presented of *A. niger* asparaginase in the presence of (combinations) of enzymes. Compared to the acrylamide level in crust of breads prepared with the mentioned enzyme or enzyme combination, the absolute acrylamide levels are lower when asparaginase is used in the presence of (combinations) of enzymes. In some cases the relative amount of acrylamide that is left is higher as a result of the lower acrylamide content in the absence of the enzyme asparaginase. The absolute acrylamide level in the presence of the enzyme combination plus asparaginase is however lower than the reference.

From Table 4 and Figure 3 it can be concluded that addition of the baking enzymes Bakezyme GOX 10,000, Bakezyme MA 10,000, Bakezyme BXP501and Pectinex to a kolibri flour-based dough will result in a lower level of acrylamide in the crust when the enzyme or enzyme combination are combined with an appropriate amount of asparaginase, compared to a reference bread with asparaginase as the sole baking enzyme.

**Claims**

1.   Use of an enzymatic composition comprising:

    a. asparaginase and;
    b. xylanase.

    in the production of a food product to decrease acrylamide levels in the food product.

2.   Use according to claim 1, wherein the food product is a flour-based, corn-based or potato-based food product.

3.   Use according to claim 1 or 2, wherein the use is in the baking industry.

4.   Method for the production of a food product involving at least one heating step, comprising adding:

    a. asparaginase and
    b. at least one hydrolyzing enzyme, wherein the hydrolyzing enzyme is a xylanase to an intermediate form of said food product in said production process whereby the asparaginase and the at least one hydrolyzing enzyme are added prior to said heating step in an amount that is effective in reducing the level of acrylamide of the food product in comparison to a food product whereto no asparaginase and hydrolyzing enzyme were added.

5.   Method according to claim 4, wherein the asparaginase and the at least one hydrolyzing enzyme are added separately.

6.   Method according to claim 4, whereby components a. and b. are added in a single composition, preferably an enzyme composition comprising:

    a. asparaginase and;
    b. xylanase.

7.   Method according to any of the claims 4 to 6, wherein the food product is a flour-based, corn based, or potato-based food product.

8. Method or use according to any one of claims 2 to 7, whereby the food product is a baked product.

9. Method according to claim 8, wherein the baked product is a baked product from wheat flour and/or flours from other cereal origin.

10. Method or use according to any one of claims 2 to 9, whereby the food product is a deep fried, toasted or roasted product.

11. Method or use according to any one of claims 2-10, wherein said intermediate form of said food product is a dough.

12. Method or use according to any one of claims 2-8, wherein said food product is made from at least one raw material that is of plant origin.

13. Method or use according to any one of claims 2 to 12, wherein the food product is bread, pastry, cake, pretzels, bagels, Dutch honey cake, cookies, gingerbread, gingercake, crispbread, French fries, pommes frites, potato chips, croquettes, corn bread, corn crisps, or corn flakes.

14. Method according to any of the claims 4 to 13, wherein the heating step is a step at which at least a part of the intermediate form of said food product is exposed to temperatures of 110°C or higher.

**Patentansprüche**

1. Verwendung einer enzymatischen Zusammensetzung, umfassend:

   a. Asparaginase; und
   b. Xylanase

   bei der Herstellung eines Lebensmittelprodukts zum Verringern des Acrylamidgehalts in dem Lebensmittelprodukt.

2. Verwendung gemäß Anspruch 1, wobei das Lebensmittelprodukt ein Lebensmittelprodukt auf Mehlbasis, Maisbasis oder Kartoffelbasis ist.

3. Verwendung gemäß Anspruch 1 oder 2, wobei die Verwendung in der Backindustrie ist.

4. Verfahren zum Herstellen eines Lebensmittelprodukts mit wenigstens einem Schritt des Erhitzens, umfassend Zugeben von:

   a. Asparaginase; und
   b. wenigstens einem hydrolysierenden Enzym, wobei das hydrolysierende Enzym eine Xylanase ist,

   zu einer Zwischenform des Lebensmittelprodukts bei dem Herstellungsverfahren, wobei die Asparaginase und das wenigstens eine hydrolysierende Enzym vor dem Schritt des Erhitzens in einer Menge zugegeben werden, die wirksam ist, um den Acrylamidgehalt in dem Lebensmittelprodukt im Vergleich zu einem Lebensmittelprodukt, zu dem keine Asparaginase und kein hydrolysierendes Enzym zugegeben wurden, zu verringern.

5. Verfahren gemäß Anspruch 4, wobei die Asparaginase und das wenigstens eine hydrolysierende Enzym getrennt zugegeben werden.

6. Verfahren gemäß Anspruch 4, wobei die Komponenten a. und b. in einer einzigen Zusammensetzung, vorzugsweise einer enzymatischen Zusammentzung umfassend:

   a. Asparaginase, und
   b. Xylanase,

   zugegeben werden.

7. Verfahren gemäß einem der Ansprüche 4 bis 6, wobei das Lebensmittelprodukt ein Lebensmittelprodukt auf Mehlbasis, Maisbasis oder Kartoffelbasis ist.

8. Verfahren gemäß einem der Ansprüche 2 bis 7, wobei das Lebensmittelprodukt ein gebackenes Produkt ist.

9. Verfahren gemäß Anspruch 8, wobei das gebackene Produkt ein gebackenes Produkt aus Weizenmehl und/oder Mehlen aus anderem Getreideursprung ist.

10. Verfahren gemäß einem der Ansprüche 2 bis 9, wobei das Lebensmittelprodukt ein frittiertes, getoastetes oder geröstetes Produkt ist.

11. Verfahren gemäß einem der Ansprüche 2 bis 10, wobei die Zwischenform des Lebensmittelprodukts ein Teig ist.

12. Verfahren gemäß einem der Ansprüche 2 bis 8, wobei das Lebensmittelprodukt aus wenigstens einem Ausgangsmaterial, das von pflanzlichem Ursprung ist, hergestellt ist.

13. Verfahren gemäß einem der Ansprüche 2 bis 12, wobei es sich bei dem Lebensmittelprodukt um Brot, Gebäck, Kuchen, Bretzeln, Bagels, holländischen Honigkuchen, Kekse, Lebkuchen, Ingwerkuchen, Knäckebrot, Pommes frites, Pommes-Frites, Kartoffelchips, Kroketten, Maisbrot, Maischips oder Cornflakes handelt.

14. Verfahren gemäß einem der Ansprüche 4 bis 13, wobei der Schritt des Erhitzens ein Schritt ist, bei dem wenigstens ein Teil der Zwischenform des Lebensmittelprodukts gegenüber Temperaturen von 110 °C oder höher exponiert wird.

**Revendications**

1. Utilisation d'une composition enzymatique comprenant :

   a. de l'asparaginase et
   b. de la xylanase

   dans la production d'un produit alimentaire pour faire baisser les niveaux d'acrylamide dans le produit alimentaire.

2. Utilisation selon la revendication 1, dans laquelle le produit alimentaire est un produit alimentaire à base de farine, de maïs ou de pomme de terre.

3. Utilisation selon la revendication 1 ou 2, dans laquelle l'utilisation se fait en boulangerie industrielle.

4. Procédé de production d'un produit alimentaire impliquant au moins une étape de chauffage, comprenant l'addition :

   a. d'asparaginase et
   b. d'au moins une enzyme hydrolysante, dans lequel l'enzyme hydrolysante est une xylanase

   à une forme intermédiaire dudit produit alimentaire dans ledit processus de production, moyennant quoi l'asparaginase et ladite ou lesdites enzymes hydrolysantes sont ajoutées avant ladite étape de chauffage en quantité efficace pour faire baisser le niveau d'acrylamide dans le produit alimentaire par comparaison avec un produit alimentaire auquel n'ont été ajoutés ni asparaginase ni enzyme hydrolysante.

5. Procédé selon la revendication 4, dans lequel l'asparaginase et ladite ou lesdites enzymes hydrolysantes sont ajoutées séparément.

6. Procédé selon la revendication 4 par lequel les composants a. et b. sont ajoutés dans une unique composition, de préférence une composition enzymatique comprenant :

   a. de l'asparaginase et
   b. de la xylanase.

7. Procédé selon l'une quelconque des revendications 4 à 6, dans lequel le produit alimentaire est un produit alimentaire à base de farine, de maïs ou de pomme de terre.

**8.** Procédé selon l'une quelconque des revendications 2 à 7, dans lequel le produit alimentaire est un produit de boulangerie.

**9.** Procédé selon la revendication 8, dans lequel le produit de boulangerie est un produit de boulangerie à base de farine de blé et/ou de farines d'autres céréales.

**10.** Procédé selon l'une quelconque des revendications 2 à 9, dans lequel le produit alimentaire est un produit frit, grillé ou rôti.

**11.** Procédé selon l'une quelconque des revendications 2 à 10, dans lequel ladite forme intermédiaire dudit produit alimentaire est une pâte.

**12.** Procédé selon l'une quelconque des revendications 2 à 8, dans lequel ledit produit alimentaire est fabriqué à partir d'au moins une matière première qui est d'origine végétale.

**13.** Procédé selon l'une quelconque des revendications 2 à 12, dans lequel le produit alimentaire correspond à du pain, une pâtisserie, un gâteau, un bretzel, un bagel, du pain aux épices hollandais, des biscuits, du pain d'épice, du pain scandinave, des frites, des chips, des croquettes, du pain de maïs, des chips de maïs ou des cornflakes.

**14.** Procédé selon l'une quelconque des revendications 4 à 13, dans lequel l'étape de chauffage est une étape au cours de laquelle au moins une partie de la forme intermédiaire dudit produit alimentaire est exposée à des températures égales ou supérieures à 110 °C.

Fig. 1

Fig. 2

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004037007 A
- WO 2003083043 A
- WO 2004030468 A
- WO 2004032648 A
- WO 2004026043 A
- WO 03083043 A
- WO 04030468 A

**Non-patent literature cited in the description**

- **TAREKE et al.** *Chem. Res. Toxicol.,* 2000, vol. 13, 517-522
- **MOTTRAM et al.** *Nature,* 2002, vol. 419, 448
- **BELITZ ; GROSCH.** Food Chemistry. Springer, 1999
- Recommended Enzyme Nomenclature. IUBMB. Academic Press Inc, 1992